# EUROPEAN PATENT APPLICATION

(11) **EP 1 816 210 A1**
(43) Date of publication of application: **08.08.2007**
(21) Application number: 05809508.4
(22) Date of filing: 22.11.2005
(51) Int. Cl.: C12Q 1/06, C12M 1/34, G01N 27/416

(54) **MICROBE COUNTING METHOD AND MICROBE COUNTING DEVICE**

(30) Priority: 24.11.2004 JP 2004338767
(71) Applicant: DAIKIN INDUSTRIES, LTD., Osaka-shi, Osaka 530-8323 (JP)
(72) Inventor: AKAMATSU, Megumi, DAIKIN INDUSTRIES LTD., Kusatsu-shi, Shiga 5258526 (JP); OKUMURA, Chiaki, DAIKIN INDUSTRIES LTD., Kusatsu-shi, Shiga 5258526 (JP); MIYAHARA, Seiichiro, DAIKIN INDUSTRIES LTD., Sakai-shi, Osaka 5918511 (JP); FUKUI, Naoki, DAIKIN INDUSTRIES LTD., Kusatsu-shi, Shiga 5258526 (JP)
(74) Representative: Goddar, Heinz J.
(86) International application number: PCT/JP2005/021474
(87) International publication number: WO 2006/057253

(57) **Abstract**

Various settings used in a process proceeding in line with a flow chart are reset (S100). An oxygen current (Inew) is measured (S101). A slope of the oxygen current is obtained (SA). A determination is made as to whether or not the slope can be considered to become stable (SB). A first measuring time is determined (SC). In order to determine the stability of the slope, the oxygen current (Inew) is sequentially measured and the slope of the oxygen current is updated until the slope is determined to become stable. When the slope is determined to become stable, the first measuring time is determined (SC). When the slope is not determined to become stable, the oxygen current (Inew) is further measured.

## Description

### Technical Field

The present invention relates to a method of calculating the population of microorganisms.

### Background Art

Various methods of calculating the population of microorganisms have been suggested. For example, methods which are adopted as official methods include a method in which bacteria are enriched and incubated in a culture medium and colonies are counted after passage of a predetermined time to thereby obtain the initial number of bacteria.

However, it takes approximately twenty hours to enrich and incubate bacteria sufficiently enough to visually check the colonies. As such, a method in which the concentration of dissolved oxygen in a culture medium which is supposed to be used for incubation of microorganisms is measured has been suggested. A measurement of the concentration of dissolved oxygen in a culture medium is achieved by measuring a current (which will hereinafter be referred to as an "oxygen current") flowing through an oxygen electrode, for example. The foregoing method is disclosed in Patent Document 1, for example. Nonetheless, according to the method disclosed in Patent Document 1, a time required for an oxygen current to reach a predetermined threshold value is measured.

Patent Document 1: Japanese Patent Application Laid-Open No. 2003-235599

### Description of the Invention

The object of the present invention is to suggest a method which allows for earlier evaluation of the population of microorganisms based on an oxygen current.

In accordance with a first aspect of a method of measuring microorganisms according to the present invention, the method sequentially measures (S101, S102) an oxygen current (Inew) which is a current flowing depending on an amount of oxygen in a culture medium containing microorganisms, the initial number of which is a target of measurement, to obtain a first measuring time (ts) required for the oxygen current to fall below a first threshold value (IP) a preset number of times (Z2), and includes the following steps of (a) through (c). Specifically, the step (a) is to obtain a time difference value (Knew) which is a difference in the oxygen current per unit time, from a measured value of the oxygen current in a predetermined measuring period (SA); the step (b) is to determine as to whether or not the time difference value obtained in the step (a) becomes stable (SB); and the step (c) is to, when the determination in the step (b) gives a positive result, set the first threshold value which depends on and is lower than a value based on values of the oxygen current (I1 through IZ+1) resulting from measurements in the latest measuring period (S114a, S114b).

In accordance with a second aspect of the method of measuring microorganisms according to the present invention, in the first aspect of the method of measuring the number of microorganisms, the first threshold value (IP) is set in the step (c) by subtracting a preset threshold value (Δ) from a value of the oxygen current (IZ+1) which has been most recently measured.

In accordance with a third aspect of the method of measuring microorganisms according to the present invention, in the second aspect of the method of measuring the number of microorganisms, the first threshold value (IP) is set in the step (c) by multiplying a value of the oxygen current (IZ+1) which has been most recently measured by a coefficient (q) which is larger than zero and less than one.

In the third aspect of the method of measuring microorganisms, the coefficient (q) is set to 0.8, preferably.

In accordance with a fourth aspect of the method of measuring microorganisms according to the present invention, the method sequentially measures an oxygen current (Inew) which is a current flowing depending on an amount of oxygen in a culture medium containing microorganisms, the initial number of which is a target of measurement, to obtain a first measuring time (tu) required for a decrease of the oxygen current to be moderated below a threshold value (E3), and includes the following steps of (a) through (c). Specifically, the step (a) is to obtain a time difference value (Knew) which is a difference in the oxygen current per unit time, from a measured value of the oxygen current in a predetermined measuring period (SA); the step (b) is to determine as to whether or not the time difference value obtained in the step (a) becomes stable (SB); and the step (c) is to, when the determination in the step (b) gives a positive result, determine the measuring time based on the latest measured measuring period if a value of the difference value (Knew) provided by a measurement after the step (b) increases to be larger than a value of the difference value (Kold) provided by a measurement in the step (b) by increments exceeding the threshold value (SC).

In accordance with a fifth aspect of the method of measuring microorganisms according to the present invention, in any of the first through fourth aspects of the method of measuring the number of microorganisms, in the step (b), the time difference value is determined to become stable by acknowledging that an absolute value (|(Knew)| of the time difference value falls within a predetermined range (E2) in a predetermined period (S108b, S109).

In accordance with a sixth aspect of the method of measuring microorganisms according to the present invention, in any of the first through fifth aspects of the method of measuring the number of microorganisms, in the step (b), the time difference value is determined to become stable by acknowledging that an absolute value of variation in the time difference value with respect to time (|Knew - Kold|/Δt) falls within a predetermined range (E1) in a predetermined period (S108a, S109).

In the sixth aspect of the method of measuring microorganisms, the predetermined range (E1) is equal to 0.6 nA/mm²/min² in terms of current density, the preset number of times (Z2) is equal to ten, and the predetermined period is long enough for the oxygen current to be measured five (Z1) times in succession, preferably.

In accordance with a seventh aspect of the method of measuring microorganisms according to the present invention, in any of the first through sixth aspects of the method of measuring the number of microorganisms, a determination as to whether or not the time difference value becomes stable is made in the step (b), after a predetermined dead time passes after the step (a).

In accordance with an eighth aspect of the method of measuring microorganisms according to the present invention, in the seventh aspect of the method of measuring the number of microorganisms, the predetermined dead time is set to 200 minutes.

In accordance with a ninth aspect of the method of measuring microorganisms according to the present invention, in any of the first through eighth aspects of the method of measuring the number of microorganisms, the method further includes a step of (d) obtaining a second measuring time (tt) required for the oxygen current to decrease to fall below a second threshold value (Id).

In accordance with a tenth aspect of the method of measuring microorganisms according to the present invention, in the ninth aspect of the method of measuring the number of microorganisms, the second threshold value (Id) is equal to 60 nA/nun²/min² in terms of current density.

In accordance with an eleventh aspect of the method of measuring microorganisms according to the present invention, in any of the first through tenth aspects of the method of measuring the number of microorganisms, the first measuring time for the culture medium which contains the microorganisms, the initial number of which is unknown, is obtained, to calculate the initial number of microorganisms which has been unknown, based on a calibration curve. The calibration curve is obtained from a relationship between the first measuring time and the initial number of microorganisms which is known, of each of the culture media which contain the microorganisms, the initial numbers of which are known and differ from each other, for the microorganisms and the culture media. The culture media, first measuring times of which are to be measured for the calibration curve, are of the same kind as the culture medium for calculating the initial number which has been unknown. However, although the unknown initial number of bacteria in the microorganisms is calculated based on the calibration curve, it is not necessarily required to form the calibration curve.

In accordance with a twelfth aspect of the method of measuring microorganisms according to the present invention, in any of the first through eleventh aspects of the method of measuring the number of microorganisms, the first measuring time is respectively obtained for each of the culture media of the same kind the initial numbers of the microorganisms in which are known and differ from each other, and the calibration curve for the microorganisms and the culture media is obtained from a relationship between the plural initial numbers of microorganisms which are known and plural of the first measuring time.

In accordance with a first aspect of an apparatus for measuring microorganisms according to the present invention, the apparatus includes: an oxygen current measuring part (201) for sequentially measuring (S101, S 112) an oxygen current (Inew) which is a current flowing depending on an amount of oxygen in a culture medium containing microorganisms, the initial number of which is a target of measurement; and an evaluating part (202) for obtaining a first measuring time (ts) required for the oxygen current to decrease to fall below a first threshold value (IP) a preset number of times (Z2). The evaluating part performs the following steps of (a) through (c). Specifically, the step (a) is to obtain a time difference value (Knew) which is a difference in the oxygen current per unit time, from a measured value of the oxygen current in a predetermined measuring period (SA). The step (b) is to determine as to whether or not the time difference value obtained in the step (a) becomes stable (SB). The step (c) is to, when the determination in the step (b) gives a positive result, set the first threshold value which depends on and is lower than a value based on values of the oxygen current (I1 through IZ+1) resulting from measurements in the latest measuring period (S 114a, S 114b).

In accordance with a second aspect of the apparatus for measuring microorganisms according to the present invention, in the first aspect of the apparatus for measuring the number of microorganisms, the first threshold value (IP) is set in the step (c) by subtracting a preset threshold value (Δ) from a value of the oxygen current (IZ+1) which has been most recently measured.

In accordance with a third aspect of the apparatus for measuring microorganisms according to the present invention, in the second aspect of the apparatus for measuring the number of microorganisms, the first threshold value (IP) is set in the step (c) by multiplying a value of the oxygen current (IZ+1) which has been most recently measured by a coefficient (q) which is larger than zero and less than one.

In the third aspect of the apparatus for measuring microorganisms, the coefficient (q) is set to 0.8, preferably.

In accordance with a fourth aspect of the apparatus for measuring microorganisms according to the present invention, the apparatus includes: an oxygen current measuring part (201) for sequentially measuring an oxygen current (Inew) which is a current flowing depending on an amount of oxygen in a culture medium containing microorganism, the initial number of which is a target of measurement; and an evaluating part (202) for obtaining a first measuring time (tu) required for a decrease of the oxygen current to be moderated below a threshold value (E3). The evaluating part performs the following steps of (a) through (c). Specifically, the step (a) is to obtain a time difference value (Knew) which is a difference in the oxygen current per unit time, from a measured value of the oxygen current in a predetermined measuring period (SA). The step (b) is to determine as to whether or not the time difference value obtained in the step (a) becomes stable (SB). The step (c) is to, when the determination in the step (b) gives a positive result, determine the measuring time based on the latest measured if a value of the difference value (Knew) provided by a measurement after the step (b) increases to be larger than a value of the difference value (Kold) provided by a measurement in the step (b) by increments exceeding the threshold value (SC).

In accordance with a fifth aspect of the apparatus for measuring microorganisms according to the present invention, in any of the first through fourth aspects of the apparatus for measuring the number of microorganisms, the time difference value is determined to become stable by acknowledging that an absolute value (|(Knew)| of the time difference value falls within a predetermined range (E2) in a predetermined period (S 1 08b, S109) in the step (b).

In accordance with a sixth aspect of the apparatus for measuring microorganisms according to the present invention, in any of the first through fifth aspects of the apparatus for measuring the number of microorganisms, in the step (b), the time difference value is determined to become stable by acknowledging that an absolute value of variation in the time difference value with respect to time (|Knew - Kold|/Δt) falls within a predetermined range (E1) in a predetermined period (S108a, S109).

In the sixth aspect of the apparatus for measuring microorganisms, the predetermined range (E1) is equal to 0.6 nA/mm²/min² in terms of current density, the preset number (Z2) of times is equal to ten, and the predetermined period is long enough for the oxygen current to be measured five (Z1) times in succession, preferably.

In accordance with a seventh of the apparatus for measuring microorganisms according to the present invention, in any of the first through sixth aspects of the apparatus for measuring the number of microorganisms, in the step (b), a determination as to whether or not the time difference value becomes stable is made after a predetermined dead time passes after the step (a).

In accordance with an eighth aspect of the apparatus for measuring microorganisms according to the present invention, in the seventh aspect of the apparatus for measuring the number of microorganisms, the predetermined dead time is set to 200 minutes.

In accordance with a ninth aspect of the apparatus for measuring microorganisms according to the present invention, in any of the first through eighth aspects of the apparatus for measuring the number of microorganisms, the evaluating part (201) further performs a step of (d) obtaining a second measuring time (tt) required for the oxygen current to decrease to fall below a second threshold value (Id).

In accordance with a tenth aspect of the apparatus for measuring microorganisms according to the present invention, in the ninth aspect of the apparatus for measuring the number of microorganisms, the second threshold value (Id) is equal to 60 nA/mm²/min² in terms of current density.

In accordance with an eleventh aspect of the apparatus for measuring microorganisms according to the present invention, in any of the first through tenth aspects of the apparatus for measuring the number of microorganisms, the first measuring time for one of the culture media which contains the microorganisms, the initial number of which is unknown, is obtained, to calculate the initial number of microorganisms which has been unknown, based on a calibration curve. The calibration curve is obtained from a relationship between the first measuring time and the initial number of microorganisms which is known, of each of the culture media which contain the microorganisms, the initial numbers of which are known and differ from each other, for the microorganisms and the culture media. The culture media, the first measuring times of which are to be measured based on the calibration curve, are of the same kind as the culture medium for calculating the initial number which has been unknown. However, although the unknown initial number of bacteria in the microorganisms is calculated based on the calibration curve, it is not necessarily required to form the calibration curve.

In accordance with a twelfth aspect of the apparatus for measuring microorganisms according to the present invention, in any of the first through eleventh aspects of the apparatus for measuring the number of microorganisms, the first measuring time is respectively obtained for each of the culture media of the same kind the initial numbers of the microorganisms in which are known and differ from each other, and the calibration curve for the microorganisms and the culture media is obtained from a relationship between the plural of initial numbers of microorganisms which are known and plural of the first measuring time.

In the first aspect of each of the apparatus for, and the method of, measuring the number of microorganisms according to the present invention, a time required for a process for obtaining the initial number of microorganisms by measuring the current flowing depending on the amount of oxygen in the culture medium is shortened.

In the second aspect of each of the apparatus for, and the method of, measuring the number of microorganisms according to the present invention, the first threshold value in the step (c) is determined.

In the third aspect of each of the apparatus for, and the method of, measuring the number of microorganisms according to the present invention, in obtaining the first measuring time using the first threshold value determined in the second aspect, the probability of coincidence with results given by a conventional process is increased.

In the fourth aspect of each of the apparatus for, and the method of, measuring the number of microorganisms according to the present invention, a time required for a process for obtaining the initial number of microorganisms by measuring the current flowing depending on the amount of oxygen in the culture medium is shortened.

In the fifth aspect of each of the apparatus for, and the method of, measuring the number of microorganisms according to the present invention, the stability of the time difference value in the step (b) is determined.

In the sixth aspect of each of the apparatus for, and the method of, measuring the number of microorganisms according to the present invention, in determining the stability of the time difference value in the fifth aspect, the probability of coincidence with results given by a conventional process is increased.

In the seventh aspect of each of the apparatus for, and the method of, measuring the number of microorganisms according to the present invention, influences of irregularities in the current at the beginning of measurement upon the determination in the step (b) are removed.

In the eighth aspect of each of the apparatus for, and the method of, measuring the number of microorganisms according to the present invention, in obtaining the first measuring time using a dead time employed in the seventh aspect, the probability of coincidence with results given by a conventional process is increased.

In the ninth aspect of each of the apparatus for, and the method of, measuring the number of microorganisms according to the present invention, even when the initial number of microorganisms is so large that the determination in the step (c) cannot be made, the initial number of microorganisms can be obtained by using the second measuring time.

In the tenth aspect of each of the apparatus for, and the method of, measuring the number of microorganisms according to the present invention, in obtaining the second measuring time employed in the ninth aspect, the probability of coincidence with results given by a conventional process is increased.

In the eleventh aspect of each of the apparatus for, and the method of, measuring the number of microorganisms according to the present invention, a time required for a process for obtaining the initial number of microorganisms by measuring the current flowing depending on the amount of oxygen in the culture medium is shortened.

In the twelfth aspect of each of the apparatus for, and the method of, measuring the number of microorganisms according to the present invention, the calibration curve used in calculating the initial number of microorganisms in the eleventh aspect is formed.

Therefore, the object, features, aspects and advantages of the present invention will become more apparent from the following detailed description of the present invention and the accompanying drawings.

### Brief Description of Drawings

[Fig. 1] is a graph exemplifying a relationship between a measuring time and an oxygen current by using the initial number of bacteria as a parameter.
[Fig. 2] is a flow chart showing a part of a method of measuring the number of microorganisms according to a first preferred embodiment of the present invention.
[Fig. 3] is a flow chart exemplifying details of a step SA.
[Fig. 4] is a flow chart exemplifying details of a step SB.
[Fig. 5] is a flow chart exemplifying details of another example of the step SB.
[Fig. 6] is a flow chart exemplifying details of a step SC.
[Fig. 7] is a flow chart exemplifying details of another example of the step SC.
[Fig. 8] is a graph showing an outline of a process for obtaining a first measuring time by measuring an oxygen current.
[Fig. 9] is a flow chart of the step SA according to a second preferred embodiment of the present invention.
[Fig. 10] is a graph showing a first instance in which a value of an oxygen current rapidly decreases.
[Fig. 11] is a graph showing a second instance in which a value of an oxygen current rapidly decreases.
[Fig. 12] is a graph showing a third instance in which a value of an oxygen current rapidly decreases.
[Fig. 13] is a flow chart showing a part of a method of measuring the number of microorganisms according to a third preferred embodiment of the present invention.
[Fig. 14] is a flow chart showing details of the step SA according to the third preferred embodiment of the present invention.
[Fig. 15] is a flow chart exemplifying details of the step SA according to the third preferred embodiment of the present invention.
[Fig. 16] is a flow chart exemplifying details of the step SC according to the third preferred embodiment of the present invention.
[Fig. 17] is a flow chart exemplifying details of the step SC according to a fourth preferred embodiment of the present invention.
[Fig. 18] is a block diagram showing a structure of a microorganism calculator to which the present invention is applicable.

### Best Mode for Carrying Out the Invention

### Basic Concept of the Present Invention

Prior to detailed description of preferred embodiments of the present invention, the basic concept of the present invention will be described. The basic concept described below is within the scope of the present invention as well, of course. Though the following description will be made by taking bacteria as one example of microorganisms, the other kinds of microorganisms can be dealt with in the same way as described below. Also, since a value of an oxygen current and a threshold value related thereto are normalized by an area of an oxygen electrode to be described, the values should be dealt with as a current density, to be exact. However, the values will be dealt as current values in the following description for purposes of facilitating discussions.

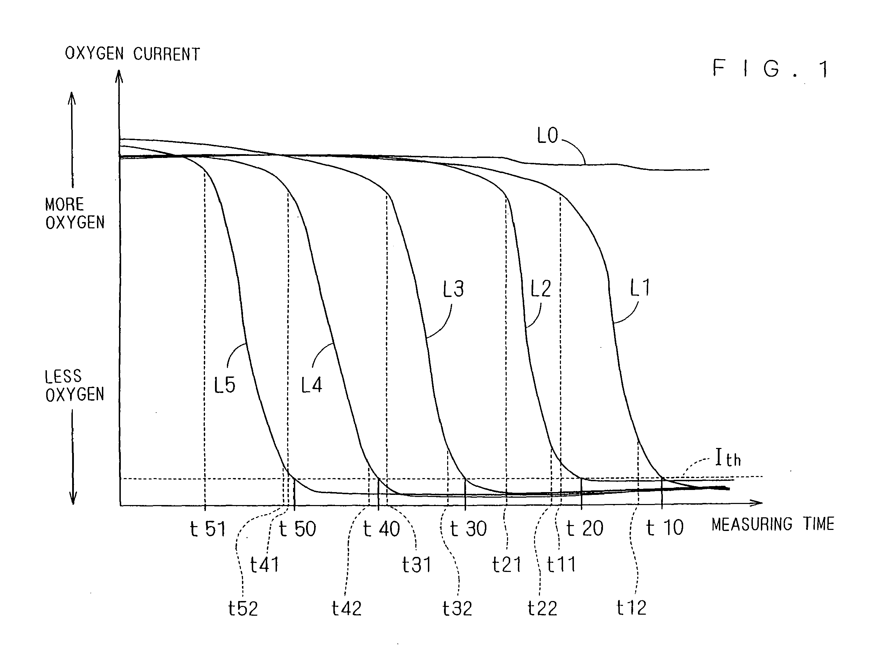

Fig. 1 is a graph exemplifying a relationship between a measuring time and an oxygen current by using the initial number of bacteria as a parameter. Since enrichment and incubation are caused in the course of a measuring time, an amount of dissolved oxygen decreases, so that an oxygen current correspondingly decreases. A graph L0 exemplifies a case in which the initial number of bacteria is zero, and graphs L1, L2, L3, L4, and L5 exemplify cases in which the initial numbers of bacteria are 101, 102, 103, 104, and 105 (CFU/ml), respectively.

Conventionally, a threshold value Ith for an oxygen current was set to a low value. Then, respective measuring times required for the graphs L1, L2, L3, L4, and L5 to reach the threshold value Ith (measuring times t10, t20, t30, t40, and t50, respectively) were obtained. Thus, a calibration curve can be obtained based on the measuring times t10, t20, t30, t40, and t50 and the initial numbers of bacteria 101, 102, 103, 104, and 105 (CFU/ml) which have been already known. Accordingly, even if the initial number of bacteria is unknown, the initial number of bacteria can be calculated based on the measuring time required to reach the threshold value Ith and the calibration curve.

However, as is shown in the graphs L1, L2, L3, L4, and L5, while an oxygen current decreases with the passage of a measuring time, a slope of the decrease thereof is small at the beginning. After a certain time passes, the slope of the decrease increases, and thereafter, the slope of the decrease further increases. Therefore, by obtaining a measuring time at a point in time when the slope of the graph of an oxygen current with respect to time (in other words, a difference in oxygen current per unit time, which will hereinafter be simply referred to as a "slope of an oxygen current") varies (such measuring time will be hereinafter referred to as a "slope varying time"; as for the graphs L1, L2, L3, L4, and L5, a slope varying time corresponds to each of measuring times t11, t21, t31, t41, and t51 at which the slope of the decrease transits from small to large, or corresponds to each of measuring times t12, t22, t32, t42, and t52 at which the slope of the decrease transits from large to small), it is possible to form a calibration curve by using the measuring time and the initial number of bacteria which is known.

Then, the initial number of bacteria can be calculated based on the described calibration curve and a slope varying time in a case where the initial number of bacteria is unknown. Furthermore, the slope varying time t1 or t12 is shorter than the measuring time t10, the slope varying time t21 or t22 is shorter than the measuring time t20, the slope varying time t31 or t32 is shorter than the measuring time t30, the slope varying time t41 or t42 is shorter than the measuring time t40, and the slope varying time t51 or t52 is shorter than the measuring time t50. Accordingly, by obtaining a slope varying time of an oxygen current, it is possible to earlier calculate the initial number of bacteria. This is the basic concept of the present invention.

However, to visually check variation in a slope of an oxygen current would hinder automation of operations. Also, it is impossible to acknowledge a slope varying time by visual check unless a measurement for a graph is performed not only prior to passage of a slope varying time but also after passage of a slope varying time. As such, a measuring time cannot be easily shortened.

In view of the foregoing, an expedient which eliminates the need of careful observation of variation in a slope of an oxygen current is employed in first through third preferred embodiments which will be described later. Specifically, when a slope of an oxygen current becomes stable after the slope varying time t11, t21, t31, t41, or t51, a first threshold value is determined based on a graph obtained so far. Then, a calibration curve is formed based on a measuring time required for an oxygen current to decrease to fall below the first threshold value more than one time (which will hereinafter be referred to as a "first measuring time"), and the known initial number of bacteria.

Also, according to a fourth preferred embodiment, after a slope of an oxygen current becomes stable, a calibration curve is formed based on a first measuring time (which corresponds to the slope varying times t12, t22, t32, t42, or t52) which is required for an absolute value of the slope to decrease, in other words, a first measuring time at which a graph is inflected (and thus, is different from a first measuring time according to the first through third preferred embodiments), and the initial number of bacteria which is already known.

After a calibration curve is formed, the initial number of bacteria which is unknown can be calculated based on the foregoing calibration curve and the first measuring time.

### First Preferred Embodiment

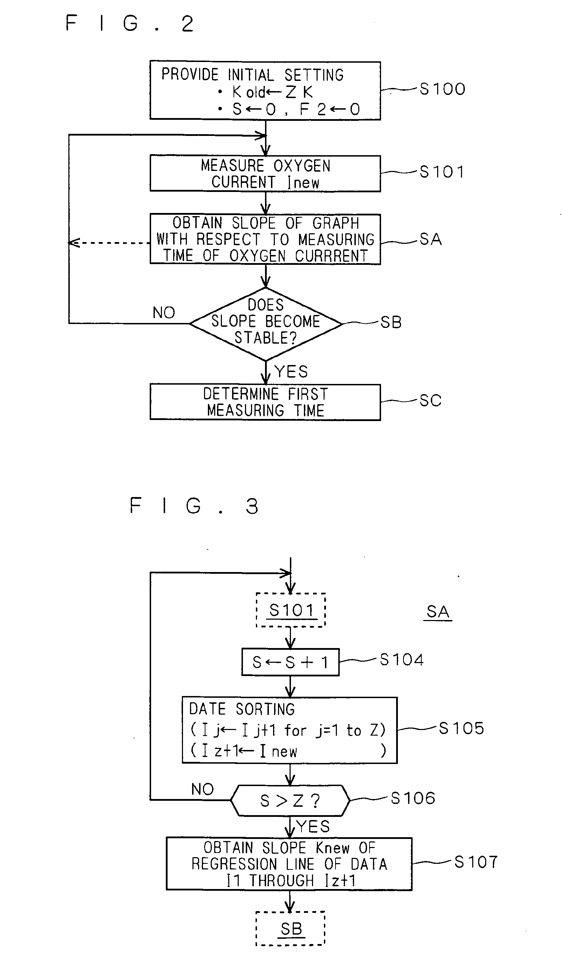

Fig. 2 is a flow chart showing a part of a method of measuring the number of microorganisms according to the first preferred embodiment of the present invention. Each of counters S and F2 which are used in a process which proceeds in line with the flow chart and a slope Kold of an oxygen current are set to an initial value (i.e., reset in a step S100). A step S101 is to measure an oxygen current Inew. A step SA is to obtain a slope of an oxygen current. A step SB is to determine whether or not the slope thus obtained can be considered to become stable. A step SC is to determine a first measuring time.

In the step SB, in order to determine whether or not a slope becomes stable, the oxygen current Inew is sequentially measured, and the slope of the oxygen current is updated until the slope is determined to become stable. As such, two destinations of the step SB are provided. When the slope is determined to become stable, the process proceeds from the step SB to the step SC. Otherwise, when the slope is not determined to become stable, the process returns back to the step S101, where the oxygen current Inew is further measured.

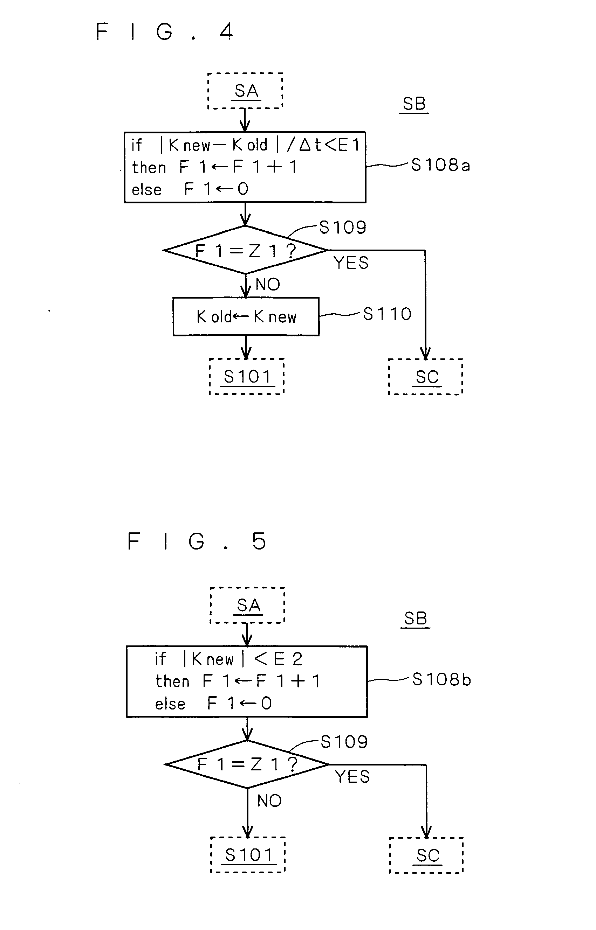

Fig. 3 is a flow chart exemplifying details of the step SA. First, the counter S is incremented by one in a step S104. The counter S is set to a value of zero in the step S 100 until the first performance of the step S 104. As a result of the first performance of the step S104, the value of the counter S becomes equal to one.

Next, data sorting of an oxygen current is performed in a step S105. More specifically, values of data 12 through IZ+1 are substituted into data 11 through IZ, and subsequently, the latest oxygen current Inew which are provided as a result of measurements in the step S101 is substituted into data IZ+1.

Then, a determination is made as to whether or not the counter S is greater than a value Z in a step S 106. When the determination made in the step S 106 gives a negative result, the process returns back to the step S101, where the next oxygen current Inew is provided by a measurement. Thus, the steps S101 through S105 are repeatedly performed until the determination made in the step S106 gives a positive result, so that Z values of an oxygen current which are put in chronological order are set in the data I1 through IZ. In a case where the counter S is equal to or smaller than the value Z, the process exceptionally returns back to the step S101 from the step SA. Such branching is indicated by a broken line in Fig. 2.

When the counter S becomes greater than the value Z, the process proceeds to a step S107. In the step S107, a slope Knew of a regression line of the data I1 through IZ+1 is obtained. If the oxygen current Inew is measured at regular intervals T, for example, in the step S101, a measuring time in which the step S101 is performed is not particularly required in order to perform the step S107. The slope Knew can be obtained by using the least squares method, for example.

A step which can replace the step S107 and can be performed more easily than the step S107 includes obtaining a difference between pieces of the data (IZ+1-I1) and dividing the difference by Z T. In any case, a period required for acquiring the data I1 through IZ+1 (Z T, for example) is defined as a measuring period, and then, the slope Knew is obtained from measured values of the oxygen current in the measuring period, to be provided as a time difference value which is a difference in oxygen current per unit time.

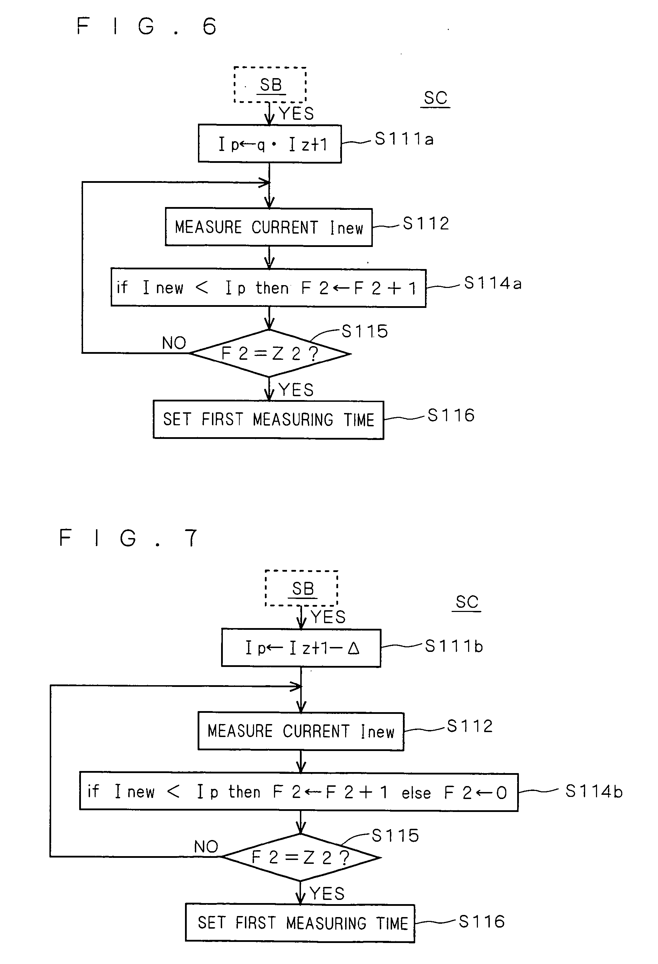

After the step S107 is performed, the process proceeds to the step SB. Fig. 4 is a flow chart exemplifying details of the step SB. First, a determination is made as to whether or not variation in a slope of an oxygen current falls within a predetermined tolerance in a step S 108a. More specifically, a determination is made as to whether or not a value obtained by dividing an absolute value of a difference between the slope Knew obtained in the step S 107 and the slope Kold by a time interval Δt between the previous measuring time of the oxygen current and the present measuring time of the oxygen current is smaller than a preset value E1. Then, when the foregoing determination gives a positive result, the counter F1 is incremented by one. On the other hand, when the foregoing determination gives a negative result, the value of the counter F1 is set to a value of zero. For the time interval Δt, the regular interval T which has been referred to above is employed, for example.

The slope Kold is set to a value ZK in the step S100 until the first performance of the step S108a. For the value ZK, a value which is greater than the slope Knew obtained in an ordinary measurement and exceeds a product of the preset value E1 and the time interval Δt is employed. Accordingly, the value of the counter F1 is certainly equal to zero as a result of the first performance of the step S108a.

Thereafter, a determination is made as to whether or not the counter F1 reaches a preset value Z1 in a step S109. In the first performance of the step S109, since the counter F1 indicates a value of zero as described above, the determination gives a negative result, so that the process proceeds to a step S 110. In the step S 110, the slope Knew which has been obtained under the present circumstances is employed as the slope Kold. Then, the process returns back to the step S101, where the slope Knew is newly obtained by performing the step SA again. Therefore, it is preferable that the above-noted interval T is longer than a sum of respective processing times of the steps SA and SB.

As a result of the above-described operations, the slope Knew which is updated by the latest oxygen current Inew which is sequentially measured is compared with the slope Kold which has been obtained immediately before the latest in the step S108a. Also, in view of the operations in the steps S 1 08a and S109, the process proceeds to the step SC after variation in a slope of an oxygen current which is repeatedly updated falls within a range of the preset value E1 Z1 times in succession.

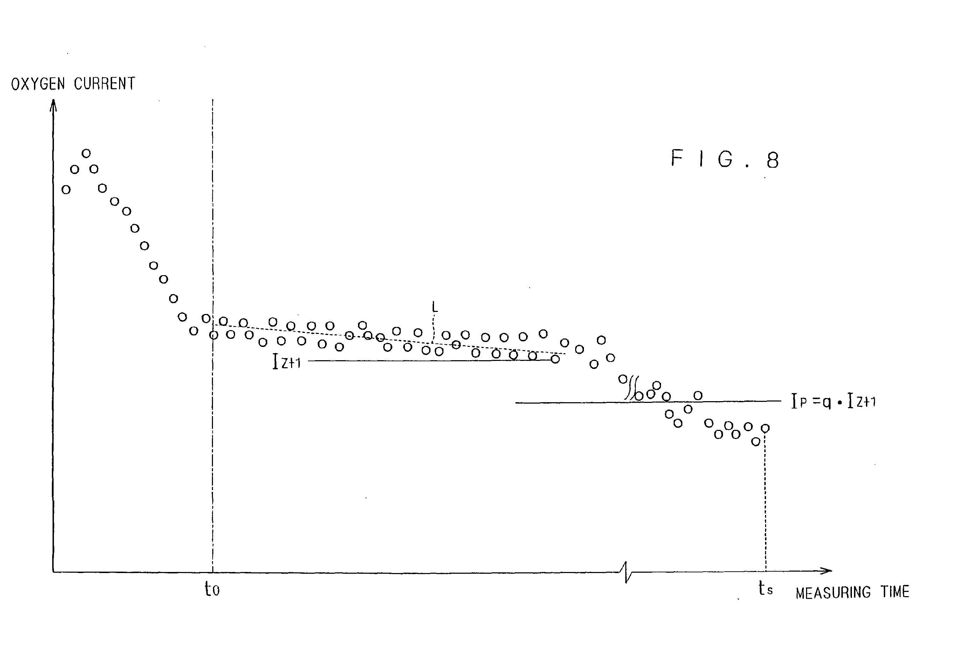

Fig. 5 is a flow chart exemplifying details of another example of the step SB. According to the example in Fig. 5, a flow chart in which the step S108a is replaced by a step S 1 08b and the step S 110 is removed as compared to the flow chart in Fig. 4 is employed.

In the step S108b, a determination is made as to whether or not an absolute value of the slope Knew is smaller than a preset value E2. Then, when the determination gives a positive result, the counter F1 is incremented by one. On the other hand, when the determination gives a negative result, the counter F1 becomes equal to a value of zero.

Fig. 6 is a flow chart exemplifying details of the step SC. In the step SC, a first threshold value IP is determined first in a step S111a. More specifically, a value which is q (0 < q < 1) times as great as the data IZ+1 when the process proceeds from the step SB to the step SC is employed as the first threshold value IP. Then, an oxygen current is again measured in a step S 112, to obtain the oxygen current Inew.

It is easy to perform the step S 112 in the same way as the step S101, and thus it is preferable that the steps S 112 and S101 are performed in conjunction with each other by measuring an oxygen current at the intervals T. In such case, the regular interval T is preferably longer than a time obtained by adding a processing time of the step S111 a to the sum of the respective processing times of the steps SA and SB. In such a preferable case as described above, a value obtained by multiplying the latest oxygen current Inew by a coefficient q can be employed as the first threshold value IP in the step S 111a.

However, in general cases, it is sufficient that the first threshold value IP depends on a value based on an oxygen current in the latest measuring period for measuring a slope because the stability of a slope is determined based on a slope which has been most recently calculated. For example, the first threshold value IP can be determined based on an average value of oxygen currents which are provided in the latest measuring period. A value obtained by multiplying the average value by the coefficient q can be employed, for example.

Then, in a step S114a, a determination is made as to whether or not the oxygen current Inew measured in the step S112 is smaller than the first threshold value IP. When the determination gives a positive result, the value of the counter F2 increases by one. Since zero is set in the counter F2 as an initial value in the step S100, the value of the counter F2 before the first performance of the step S114a is zero.

Thereafter, the process proceeds to the step S115, where a determination is made as to whether or not the value of the counter F2 reaches the preset value Z2 (> 1). In the first performance of the step S114a, since the value of the counter F1 is either zero or one, the determination gives a negative result. Accordingly, the process returns back to the step S112, where the oxygen current Inew is again measured. Therefore, the interval T is preferably longer than the sum of the respective processing times of the steps S114a and S115.

The step S114a is different from the steps S108a and S108b in that the counter F2 is not reset when a determination gives a negative result. Thus, if the oxygen current Inew which is sequentially updated in the step S112 becomes smaller than the first threshold value IP, regardless of whether or not consecutive, Z2 times, the process proceeds to the step S116. In the step S116, a measuring time of the last performance of the step S 112, for example, is employed as a first measuring time.

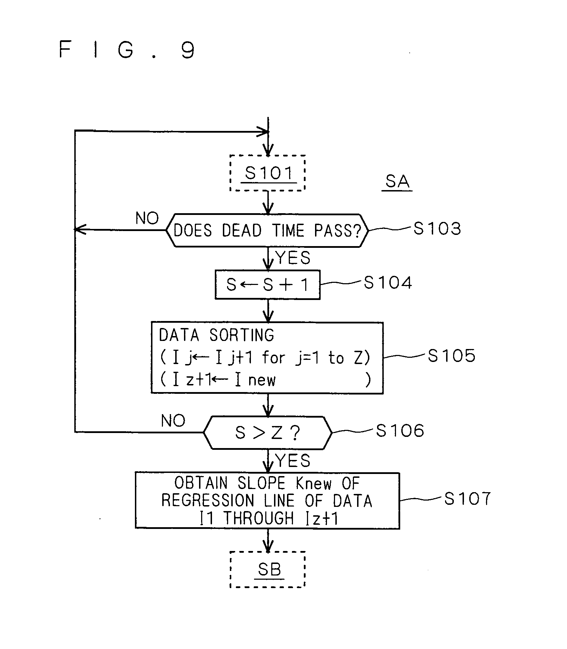

Fig. 7 is a flow chart exemplifying details of another example of the step SC. In the example in Fig. 7, the steps S111 a and S 114a are replaced by steps S111 b and S114b, respectively. In the step S111b, a value obtained by subtracting a current reduction threshold value Δ from the data IZ+1 provided when the process proceeds from the step SB to the step SC is employed as the first threshold value IP. The first threshold value IP depends on a value based on an oxygen current measured in the latest measuring period in general cases, as described above. Thus, a value obtained by subtracting the current reduction threshold value Δ from an average value of the measured oxygen currents provided in the latest measuring period can alternatively be employed as the first threshold value IP.

Further, the counter F2 is reset to zero unless a condition of Inew < IP is satisfied in the step S114b like the steps S 108a and S108b. In other words, reaching the step S 116 is caused only after the foregoing condition is satisfied Z2 times in succession.

A first measuring time is obtained in the forgoing manner. As such, in order to form a calibration curve, a first measuring time in a case where the initial number of bacteria is known is measured, first, as described above. After a calibration curve is obtained, it is possible to calculate the initial number of bacteria based on the calibration curve by measuring a first measuring time even if the initial number of bacteria is unknown.

As the values Z, Z1, and Z2, values of 30, 5, and 10 can be respectively employed, for example. Also, one (minute) and one (minute) can be respectively employed as the intervals T and Δt, and 0.8 and 30 nA/mm² can be respectively employed as the coefficient q and the current reduction threshold value Δ. Further, 103 (nA/mm²/min) can be employed as the value ZK, and 0.6 (nA/mm²/min²) and 1.2 (nA/mm²/min²) can be respectively employed as the preset values E1 and E2. The above-described flow chart in a case where such specific values as cited above are employed can be described as below.

First, an oxygen current is measured every minute. Then, for data of the oxygen current for 30 consecutive minutes, a slope of a regression line is obtained. The slope is updated each time the oxygen current is measured, and the stability of the slope is determined.

According to one of methods of determining stability (refer to Fig. 4), for example, a slope is not determined to be stable when the slope is updated and an absolute value of variation in the slope exceeds 0.6 (nA/mm²/min²). A slope is determined to be stable only after an absolute value of variation in the slope falls within the range of 0.6 (nA/mm²/min ²) five times in succession.

According to another method of determining stability (refer to Fig. 5), a slope is not determined to be stable when an absolute value of the slope exceeds 1.2 (nA/mm²/min). A slope is determined to be stable only after an absolute value of the slope falls within the range of 1.2 (nA/mm²/min) five times in succession.

Then, a first threshold value is determined based on the latest oxygen current when the slope is determined to be stable.

According to one of methods of setting a first threshold value (refer to Fig. 6), for example, a value obtained by multiplying the latest oxygen current by 0.8 is set as a first threshold value. Alternatively, according to another method of setting a first threshold value (refer to Fig. 7), a value obtained by subtracting 30 (nA/mm²) from the latest oxygen current is set as a first threshold value.

Thereafter, when an oxygen current falls below a first threshold value ten times, it is considered that a slope varying time has passed, so that a first measuring time is obtained.

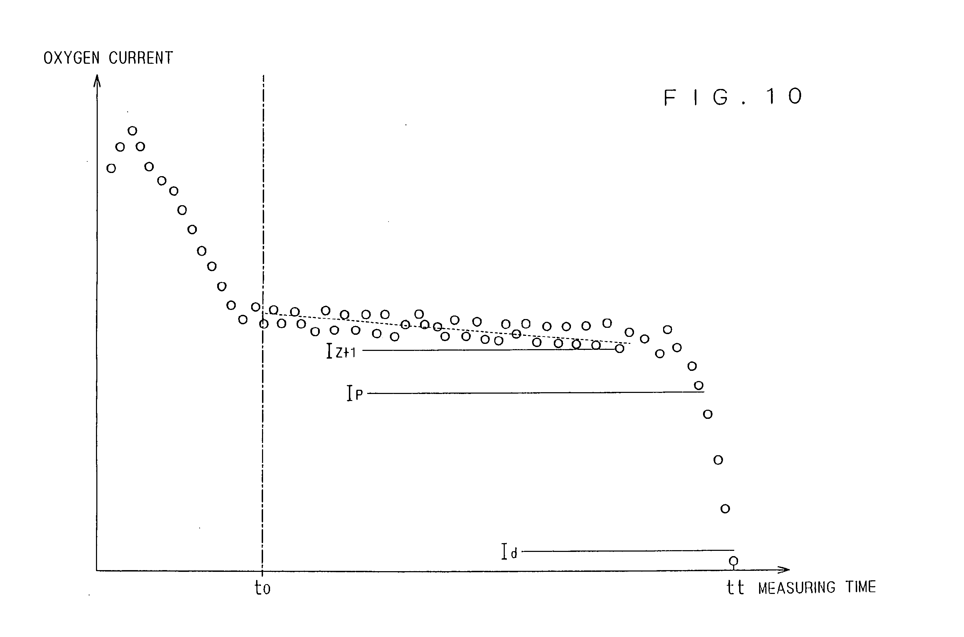

Fig. 8 is a graph showing an outline of a process for obtaining a first measuring time by measuring an oxygen current. In the graph, a horizontal axis and a vertical axis represent a measuring time and an oxygen current, respectively. A measured value of an oxygen current is represented by a white circle, and a value of the data IZ+1 provided at a time of the last performance of the step SA is represented by a transverse line. Also, for the purposes of easier understanding of the graph, a regression line L for the data I1 through IZ+1 at a time of the last performance of the step SA is shown. What is necessary to the steps SA and SB is not the regression line L, but the slope Knew thereof, as a matter of course.

Also the first threshold value IP is represented by a transverse line. In the present example, ten is set as the value Z2, for example, and a measuring time when a measured value of an oxygen current falls below the first threshold value IP ten times is employed as a first measuring time ts.

### Second Preferred Embodiment

As shown in Fig. 8, significant variation occurs before a slope becomes stable at the beginning of a measuring time in some cases. This not only results in unnecessary repetition of the operations of the step SB, but also raises the possibility of erroneously detecting as stable. Accordingly, it is preferable to include a dead time t0 in which performance of the steps which are supposed to be performed after the step SA is forbidden at the beginning of a measuring time.

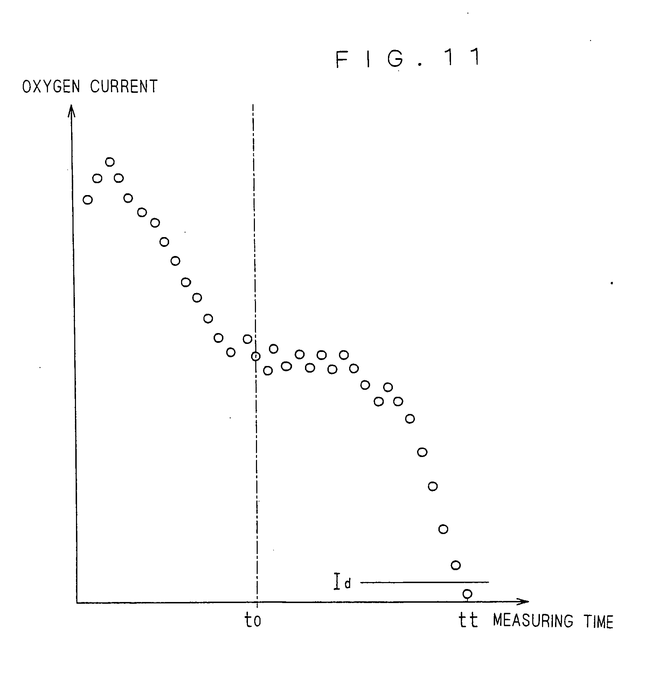

Fig. 9 is a flow chart of a process for the step SA, which takes into account inclusion of a dead time. In Fig. 9, a step S103 is added before the step S 104, as compared to the flow chart in Fig. 3. In the step S103, a determination is made as to whether or not a dead time has passed. When it is not determined that a dead time has passed, returning to the step S101 is caused. On the other hand, when it is determined that a dead time has passed, proceeding to the step S 104 is caused.

As a result, it is possible to remove influences of irregularities in a waveform of an oxygen current which are caused at the beginning of a measuring time, to thereby avoid an erroneous determination regarding a slope of a regression line of data of an oxygen current, and thus, avoid an erroneous determination regarding a first measuring time.

### Third Preferred Embodiment

In some cases, a value of an oxygen current rapidly decreases, depending on a result of a determination regarding an oxygen current. Figs. 10 through 12 are graphs for showing various instances of a rapid decrease of a value of an oxygen current.

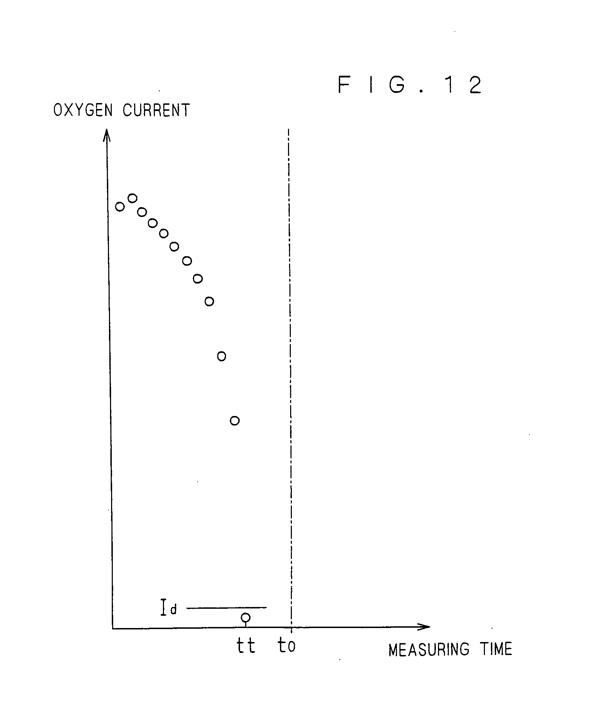

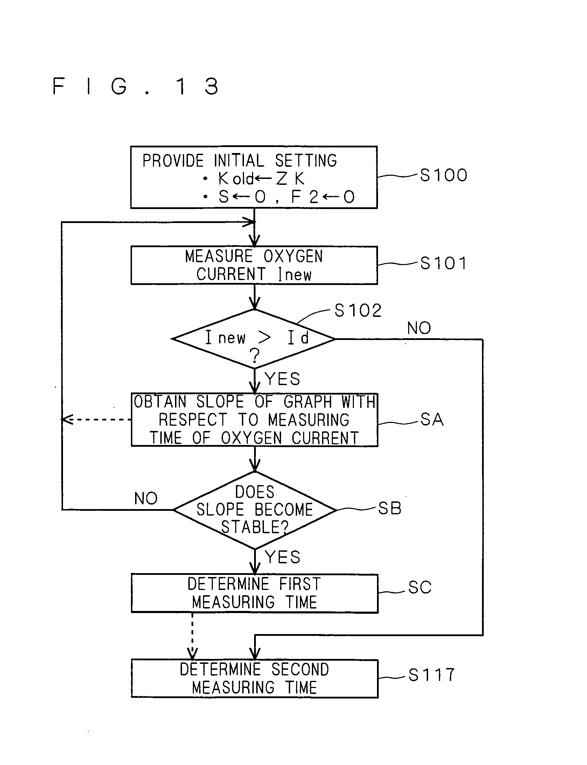

In a first instance shown in Fig. 10, after a slope of an oxygen current becomes stable, the oxygen current decreases to be un-measurable before the oxygen current falls below the first threshold value IP Z2 times. In a second instance shown in Fig. 11, an oxygen current decreases to be un-measurable before the slope thereof becomes stable. In a third instance shown in Fig. 12, an oxygen current decreases to be un-measurable as early as in a dead time. Thus, in order to allow formation of a calibration curve and calculation of the initial number of bacteria which is unknown also in the above-noted instances, a point in time at which the value of the oxygen current falls below the second threshold value Id, if it occurs, is regarded as a second measuring time tt. A second measuring time is dealt with in the same way as a first measuring time, and thus, is used for formation of a calibration curve and calculation of the initial number of bacteria which is unknown.

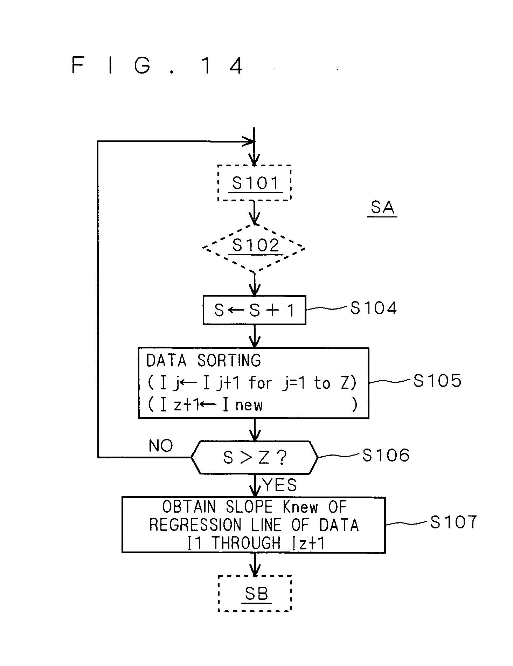

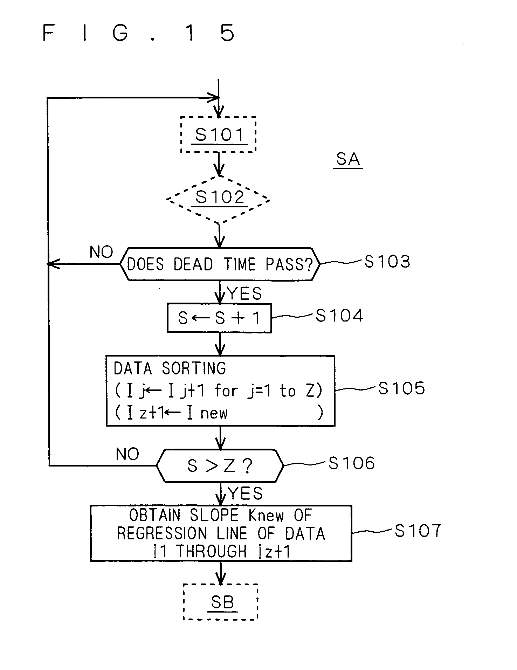

Fig. 13 is a flow chart showing a part of a method of measuring the number of microorganisms according to the third preferred embodiment of the present invention, and corresponds to Fig. 2. Also, Figs. 14 and 15 are flow charts showing details of a process for the step SA according to the third preferred embodiment of the present invention, and correspond Fig. 3 referred to in the first preferred embodiment and Fig. 9 referred to in the second preferred embodiment, respectively.

In each of Figs. 13 through 15, a determination is made as to whether or not the oxygen current Inew is greater than the second threshold value Id immediately after the oxygen current Inew is measured. More specifically, a step S102 is provided immediately after the step S101. As the second threshold value Id, 60 nA/mm² is employed, for example.

When the determination made in the step S102 gives a positive result, either the step S 104 as described in the first preferred embodiment (refer to Figs. 3 and 14) or the step S103 as described in the second preferred embodiment (refer to Figs. 9 and 15) is performed. On the other hand, when the determination made in the step S102 gives a negative result, the process proceeds to the step S 117, where a point in time at which such the oxygen current is measured is employed as a second measuring time.

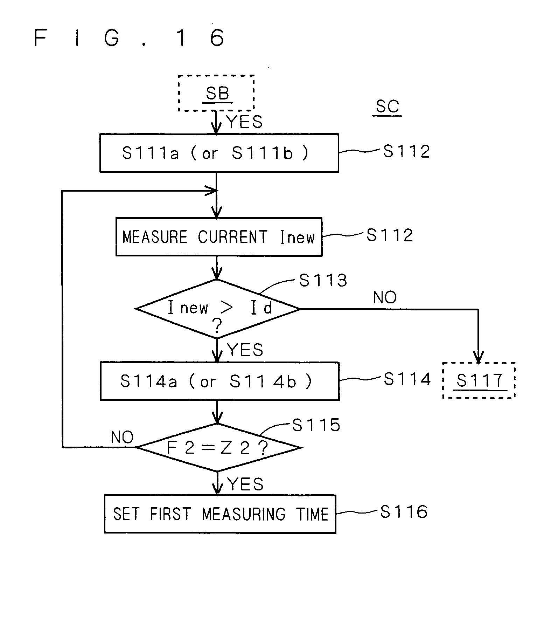

Fig. 16 is a flow chart showing details of a process for the step SC according to the third preferred embodiment of the present invention, and corresponds to each of Figs. 6 and 7 which have been referred to in the first preferred embodiment. Also in the flow chart in Fig. 16, a determination is made as to whether or not the oxygen current Inew is greater than the second threshold value Id immediately after the oxygen current Inew is measured. More specifically, a step S113 is provided immediately after the step S 112. In Fig. 16, the step S 112 is shown as integration of a step S 112a (Fig. 6) and a step S 112b (Fig. 7), and either of which is performed.

When a determination made in the step S 113 gives a positive result, either the step S114a (refer to Fig. 6) or the step S114b (refer to Fig. 7) is performed as described in the first preferred embodiment (In the Figure, the step S 112 is shown as integration of the step S 112a (Fig. 6) and the step S 112b (Fig. 7), and either of which is performed). However, when the determination made in the step S 113 gives a negative result, the process proceeds to the step S 117, where a point in time at which such the oxygen current is measured is employed as a second measuring time.

In the third preferred embodiment, the process branches out from the step SC to the step S117 in some cases, and such branching is indicated by a broken line in Fig. 13.

In the first instance shown in Fig. 10, the process branches out from the step SC to the step S 117, where a second measuring time tt is obtained, without no positive result being given by the determination made in the step S 115 (refer to Fig. 16). In the second instance shown in Fig. 11, no positive result is given by the determination made in the step S106 (refer to Figs. 14 and 15), so that the process does not proceed from the step SA to the step SB. Instead, the process proceeds from the step S 102 to the step S 117, and further proceeds in line with a branch which extends from the step SA and is indicated by a broken line in Fig. 13. In the third instance shown in Fig. 12, no positive result is given by the determination made in the step S103 in Fig. 15, so that the process does not proceeds even to the step S104, and instead proceeds from the step S 102 to the step S 117.

### Fourth Preferred Embodiment

According to this preferred embodiment, first measuring times (that are different from "first measuring times" according to the first through third preferred embodiments) t12, t22, t32, t42, or t52 at which a slope of a decrease which has been described above in the paragraphs entitled "Basic Concept of the Present Invention" transits from large to small are obtained. A first measuring time which is to be obtained according to the fourth preferred embodiment, like a first measuring time or a second measuring time according to the first through third preferred embodiments, is available for formation of a calibration curve and calculation of the initial number of bacteria which is unknown.

A dead time which has been described in the second preferred embodiment may be introduced also into this preferred embodiment, of course. Also, a second measuring time which has been described in the third preferred embodiment may be introduced into the fourth preferred embodiment. In the fourth preferred embodiment, a reference sign "tu" is used to denote a first measuring time for the purpose of distinguishing a first measuring time according to the fourth preferred embodiment from a first measuring time according to the first through third preferred embodiments.

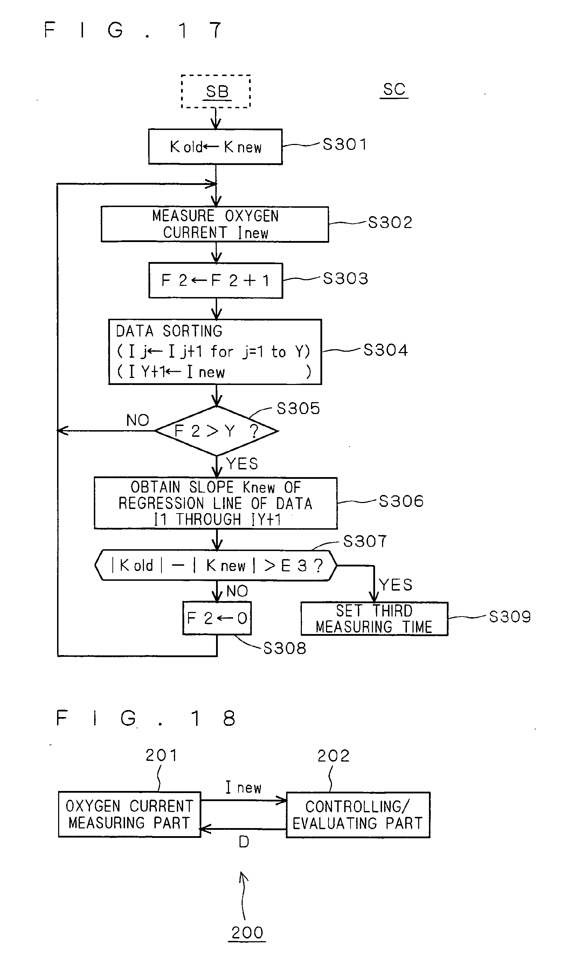

Fig. 17 is a flow chart showing details of a process for the step SC which is adapted to the fourth preferred embodiment. First, after a slope is determined to become stable in the step SB, the slope Knew which has most recently been obtained is employed as the slope Kold for the time being. Subsequently, the oxygen current Inew is newly measured in a step S302. Then, the value of the counter F2 increases by one in a step S303. Since the initial value of zero is set in the counter F2 in the step S100, the value of the counter F2 represents the number of times of performances of the step S302.

Then, data sorting of an oxygen current is performed in a step 5304 in the same manner as in the step S105, and a determination is made as to whether or not the step S302 is repeated the required number of times (Y+1) in a step S305 in the same manner as in the step S106.

The foregoing operations are the same to the operations which have been described above with reference to Fig. 3. Thus, when the determination made in the step S305 gives a positive result, Y values of an oxygen current which are put in chronological order are set in the data I1 through IZ, respectively. Thereafter, the slope Knew of a new regression line of the data I1 through IY+1 in a step S306 is obtained to be provided as a difference in oxygen current per unit time in the same manner as in the step S107.

Additionally, since the oxygen currents I1 through IZ+1 have already been obtained in the step S105 of the step SA, the result given in the step S105 may be used in the data sorting in the step S304.

Then, the size of the slope Kold employed in the step S301 and the size of the new slope Knew obtained in the step S306 are compared to each other in a step S307. Each of the slopes Kold and Knew represents a decrease of an oxygen current, and thus is a negative value. As such, a point in time at which the slope Knew becomes larger than the slope Kold by increments exceeding the preset threshold value E3 can be employed as the first measuring time tu (corresponding to the times t22, t32, t42, and t52 in Fig. 1).

For the step S307, an example in which a determination is made as to whether or not an absolute value of the slope Kold is larger than an absolute value of the slope Knew by increments exceeding the preset value E3 is shown for convenience of determination. However, it is clear that there is no need of employing an absolute value in the determination made in the step S307.

When the determination made in the step S307 gives a positive result, proceeding to the step S307 is caused, where a measuring time provided by the last performance of the step S302 is set as the first measuring time tu, for example.

When the determination made in the step S307 gives a negative result, the value of the counter F2 is reset (the value of zero is employed) in a step S308, and thereafter, returning back to the step S302 to newly obtain a slope.

The step S302 is set as a destination for returning from the step S308. In other words, a slope of an oxygen current which is determined to become stable in the step SB is employed as a standard in determining whether or not a slope of an oxygen current is moderated.

Setting the step S301 as a destination for returning from the step S308 may be thought of. In this case, the first measuring time tu is obtained based on variation in a slope of an oxygen current. However, a slope of an oxygen current gradually varies, so that the first measuring time tu in that case is longer than that in a case where the slope of an oxygen current which is determined to become stable in the step SB is employed as a standard. This lessens the effect of earlier achieving a measurement with respect to a measuring time required for an oxygen current to reach the threshold value Ith (refer to Fig. 1). Accordingly, it is preferable that not the step S301 but the step S302 is employed as a destination for the returning from the step S308 for the purpose of earlier achieving a measurement.

Alternatively, the first measuring time tu may be determined based on the latest measuring period required to obtain the slope Knew because the slope Knew is determined to become larger than the slope Kold which has been previously obtained by increments exceeding the preset threshold value E3 by the slope obtained in the latest measuring period. For example, a median of the latest measuring period may be determined as the first measuring time tu.

A first measuring time and a second measuring time obtained in the above-described various preferred embodiments are used for formation of a calibration curve in a case where the initial number of bacteria is known. Then, by measuring an oxygen current in a culture medium including bacteria, the initial number of which is unknown, based on the calibration curve thus obtained, it is possible to calculate the initial number of bacteria.

As a matter of course, it is preferable that the kind of microorganisms, the initial number of bacteria of which is known at a time of formation of a calibration curve is identical to the kind of microorganisms, the initial number of bacteria of which is unknown at a time of formation of a calibration curve, and that the kind of a culture medium including microorganisms, the initial number of bacteria of which is known at a time of formation of a calibration curve is identical to the kind of a culture medium including microorganisms, the initial number of bacteria of which is unknown at a time of formation of a calibration curve, for the purpose of improving the accuracy in measurements.

Fig. 18 is a block diagram showing a structure of a microorganism calculator 200 which is applicable to the above-described techniques for forming a calibration curve and calculating the initial number of bacteria. The microorganism calculator 200 includes an oxygen current measuring part 201 for measuring an oxygen current in a culture medium and a controlling/evaluating part 202 for controlling operations of the oxygen current measuring part 201 and evaluating an oxygen current.

The controlling/evaluating part 202 issues a command D that indicates when to measure an oxygen current, to the oxygen current measuring part 201. For example, the controlling/evaluating part 202 causes the oxygen current measuring part 201 to measure an oxygen current at the regular intervals T, or to measure an oxygen current after a dead time passes. Also, the controlling/evaluating part 202 causes the oxygen current measuring part 201 to cease a measurement of oxygen current after the first measuring time ts or tu, or the second measuring time tu, is obtained. The oxygen current measuring part 201 principally performs the steps S101 and S112, and the controlling/evaluating part 202 performs all the other steps included in the above-described flow charts, than the steps S101 and S112.

### Others

In an alternative embodiment, plural relationships between measuring times and oxygen currents are previously stored using the initial numbers of bacteria as parameters, and one of the parameters which makes the smallest difference from a measured value is defined as the initial number of bacteria which is to be obtained.

For example, the graphs L1, L2, L3, L4, and L5 shown in Fig. 1 are previously stored as current data without any modification thereto or after converting the graphs into functions. Then, measured values as indicated by white circles in Fig. 8 and Figs. 10, 11, and 12 and current data which makes the smallest difference from the foregoing current data are designated. In at least a part of measuring time, a result of summing up squares of differences between measured values and current data obtained in respective measuring times is employed as the foregoing difference, for example. Then, the initial number of bacteria which corresponds to current data which makes the smallest difference is selected as the initial number of bacteria which is obtained from the measured values.

However, in the foregoing method, graphs may be greatly deformed depending on a specimen. Thus, it is more reliable to obtain the initial number of bacteria by the method described in the above preferred embodiments.

Below, the present invention will be explained in contrast to comparative techniques. Table 1 explains the comparative techniques.

**[Table 1]**

| Detecting Condition | Dead Time (min) | Time Interval (min) | Set Current (nA/mm²) | Slope (nA/mm²/min) |
|---|---|---|---|---|
| #201 | 100 | 30 | 30 | -1.0 |
| #202 | 100 | 30 | 60 | -2.0 |
| #203 | 100 | 30 | 120 | -4.0 |
| #204 | 150 | 30 | 30 | -1.0 |
| #205 | 150 | 30 | 60 | -2.0 |
| #206 | 150 | 30 | 120 | -4.0 |

Each of detecting conditions #201 through #206 employs a point in time at which an oxygen current which is measured every minute falls below a value of a set current to a measured value of the oxygen current which was obtained 30 minutes before (time interval) five times in succession, as a detecting time. A detecting time also is considered to depend on the initial number of bacteria. A dead time is included in each of the detecting conditions #021 through #026.

For example, in the detecting condition #201, a value of a set current is set to 30 nA/mm², so that a measuring time provided when a slope of an oxygen current becomes smaller (steeper) than -30 (nA/mm²)/30 (min) = -1.0 (nA/mm²/min) five times in succession after a dead time of 100 minutes passes is employed as a detecting time.

Table 2 explains techniques for determining that a slope of an oxygen current becomes stable by acknowledging that the absolute value of the slope of the oxygen current is within a predetermined range. Such determination corresponds to the step SB described above with reference to Fig. 5.

**[Table 2]**

| Detecting Condition | Dead Time (min) | Conditions for Stability of Current Slope | | | Determination of Current Reduction | |
|---|---|---|---|---|---|---|
| | | Interval (min) | Slope (nA/mm²/min) | Number of Determinations | Threshold (nA/mm²) | Number of Determinations |
| #311 | 100 | 10 | ±1.2 | 5 | 30 | 5 |
| #312 | 100 | 10 | ±1.2 | 5 | 60 | 5 |
| #313 | 100 | 10 | ±1.2 | 5 | 90 | 5 |
| #314 | 100 | 30 | ±1.2 | 5 | 30 | 5 |
| #315 | 100 | 30 | ±1.2 | 5 | 60 | 5 |
| #316 | 100 | 30 | ±1.2 | 5 | 90 | 5 |
| #317 | 150 | 10 | ±1.2 | 5 | 30 | 5 |
| #318 | 150 | 10 | ±1.2 | 5 | 60 | 5 |
| #319 | 150 | 10 | ±1.2 | 5 | 90 | 5 |

Under detecting conditions #311 through #319, an oxygen current is measured every one minute (which corresponds to the above-noted regular interval T). A slope thereof is obtained by dividing a result of subtraction of an oxygen current which is provided ten (or thirty) minutes (which interval corresponds to a product of the value Z of the counter S and the above-noted regular interval T) from the ongoing oxygen current, by ten (or thirty) minutes. The slope thus obtained is different from the above-described slope Knew in that the least squares method is not used in obtaining the slope. At a point in time when the absolute value of the slope becomes smaller than 1.2 (nA/mm²/min) (which corresponds to the above-noted preset value E2) five (which corresponds to the above-noted preset value Z1) times in succession, it is determined that the slope of the oxygen current becomes stable. Then, at a point in time when an oxygen current having a value which is smaller than the value of the oxygen current (which corresponds to the above-noted IZ+1) provided when the slope is determined to become stable by a current reduction threshold value (which corresponds to the above-noted current reduction threshold value Δ) is observed five (which corresponds to the above-noted preset value Z2) times in succession is employed as a first measuring time. The foregoing determinations correspond to the step SC shown in Fig. 7. It is considered that also a first measuring time which is determined as described above depends on the initial number of bacteria. A dead time is included in each of the detecting conditions #311 through #319.

Under the detecting condition #311, for example, at a point in time when the absolute value of a slope of an oxygen current becomes smaller (flatter) than 1.2 (nA/mm²) five times in succession after a dead time of 100 minutes passes, the slope is determined to become stable. Then, a value which is smaller than the value of the oxygen current which is provided when the slope is determined to become stable by 30 (nA/mm²) is employed as a first threshold value IP, and a point in time when an oxygen current smaller than the first threshold value IP is observed five times in succession is employed as a first measuring time.

Table 3 explains techniques for determining that a slope of an oxygen current becomes stable by acknowledging that the absolute value of variation in the slope of the oxygen current is within a predetermined range. Such determination corresponds to the step SB described above with reference to Fig. 4.

**[Table 3]**

| Detecting Condition | Dead Time (min) | Conditions for Stability of Current Slope | | | Determination of Current Reduction | |
|---|---|---|---|---|---|---|
| | | Interval (min) | Variation in Slope (nA/mm²/min) | Number of Determinations | Threshold (nA/mm²) | Number of Determinations |
| #321 | 100 | 10 | ±3 | 5 | 30 | 5 |
| #322 | 100 | 10 | ±3 | 5 | 60 | 5 |
| #323 | 100 | 10 | ±3 | 5 | 90 | 5 |
| #324 | 100 | 30 | ±3 | 5 | 30 | 5 |
| #325 | 100 | 30 | ±3 | 5 | 60 | 5 |
| #326 | 100 | 30 | ±3 | 5 | 90 | 5 |
| #327 | 150 | 10 | ±3 | 5 | 30 | 5 |
| #328 | 150 | 10 | ±3 | 5 | 60 | 5 |
| #329 | 150 | 10 | ±3 | 5 | 90 | 5 |

A slope of an oxygen current is obtained under detecting conditions #321 through #329 in the same manner as is under the conditions #321 through #329. A slope of an oxygen current is determined to become stable at a point in time when the absolute value of variation in the slope (which corresponds to |Knew-Kold| / Δt in the step S108a in Fig. 4) becomes smaller than 3 (nA/mm²/min) five (which corresponds to the above-noted preset value Z1) times in succession. Then, at a point in time when an oxygen current having a value which is smaller than the value of the oxygen current (which corresponds to the above-noted IZ+1) provided when the slope is determined to become stable by a current reduction threshold value (which corresponds to the above-noted current reduction threshold value Δ) is observed five (which corresponds to the above-noted preset value Z2) times in succession is employed as a first measuring time. The foregoing determinations correspond to the step SC shown in Fig. 7. It is considered that also a first measuring time which is determined as described above depends on the initial number of bacteria. A dead time is included in each of the detecting conditions #321 through #329.

Under the detecting condition #321, for example, at a point in time when an absolute value of variation in a slope of an oxygen current becomes smaller (flatter) than 3 (nA/mm²/min) five times in succession after a dead time of 100 minutes passes, the slope is determined to become stable. Then, a value lower than the value of the oxygen current which is provided when the slope is determined to become stable by 30 (nA/mm²) is employed as a first threshold value IP, and a point in time when an oxygen current smaller than the first threshold value IP is observed five times in succession is employed as a first measuring time.

Tables 4, 5, and 6 show comparisons of the above-noted detecting times (in the detecting conditions #201 through #206) and the first measuring times (in the detecting conditions #311 through #319, and #312 through #329) to those in a case where a slope varying time is obtained by visually checking variation in a slope of an oxygen current (techniques by which a slope varying time is automatically obtained correspond to the fourth preferred embodiment, the results of which are indicated as "Measure" in Tables). It is noted that a condition #101 in Table 4 is a condition under which a measuring time required to reach the threshold value Ith is measured by using the conventional arts. In the condition #101, 180 nA/mm² is employed as the threshold value Ith, and a point in time when an oxygen current falls below the threshold value Ith three times is employed as a detecting time. Meanwhile, a second measuring time determined by using the second threshold value Id, which has been described above in the third preferred embodiment, is not employed.

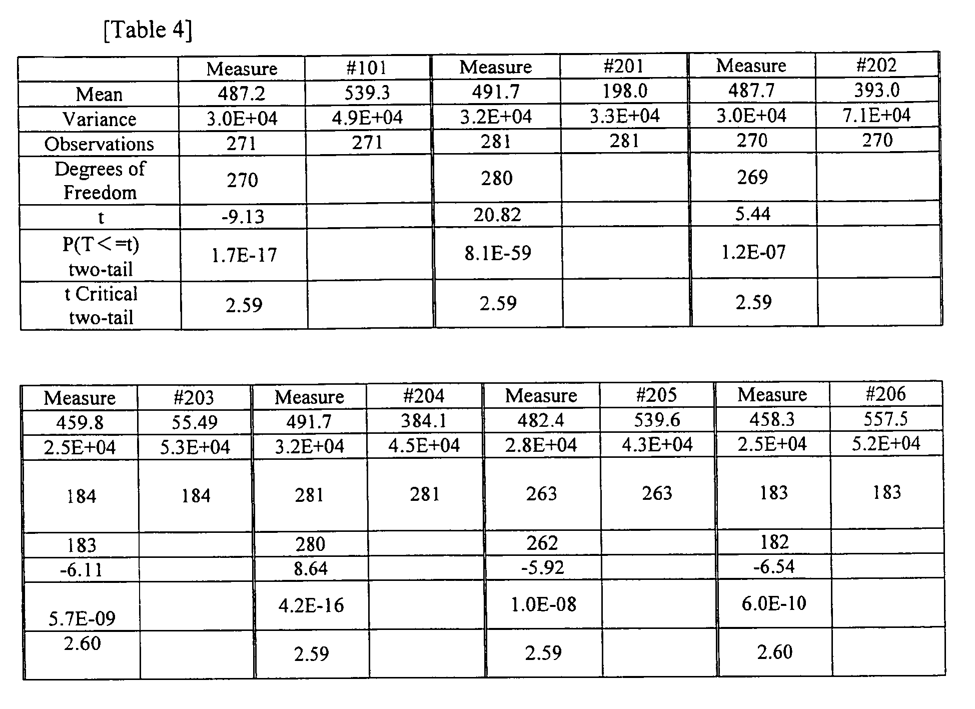

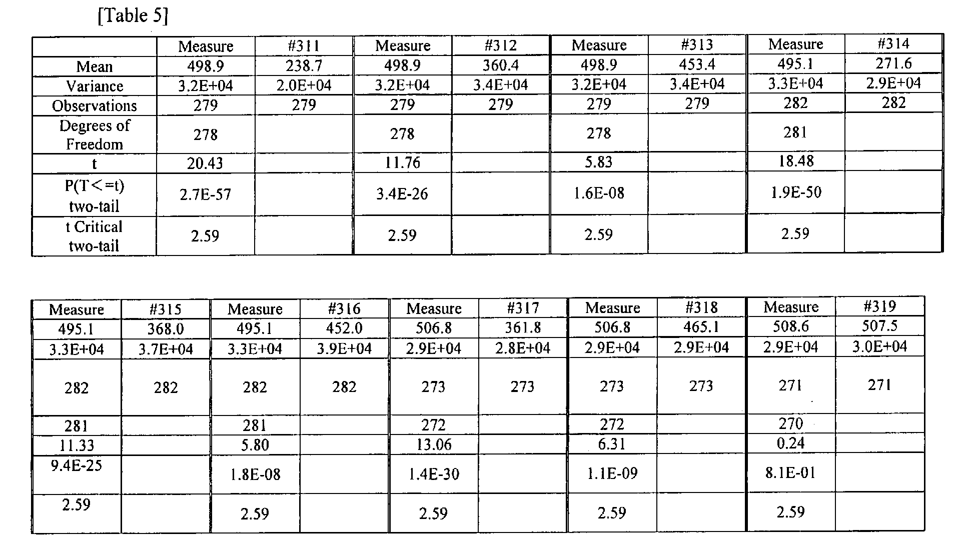

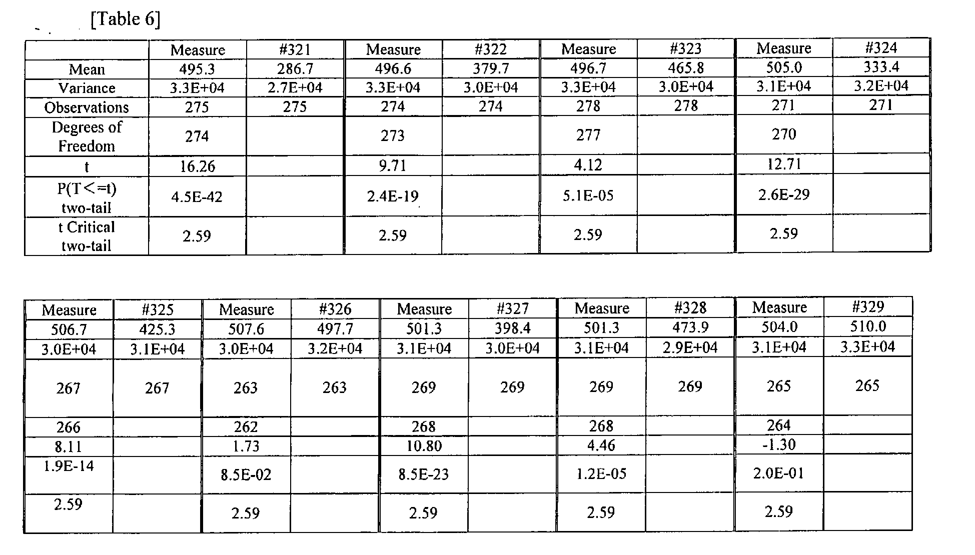

For each of the detecting conditions, a slope varying time and a measurement result (including a detecting time and a first measuring time) given under each of the detecting conditions are treated as a pair of samples, and a significant difference test (significance level of 1 %) was conducted on respective times of the pair of each sample by carrying out a one-sample t-test. As such, the degrees of freedom is equal to a value smaller than observations by one. The measures each of which is paired with any of the detecting conditions as a sample are indicated in columns respectively placed on the left side of the detecting conditions. Thus, a pair of one detecting condition and one measure which are placed adjacent to each other with a double bar being interposed therebetween are not to be compared to each other.

The total number of specimens (culture media which are measured) is 358. The observations which give measurement results in each of the detecting conditions is smaller than the total number of specimens because at least one of a measurement result (including a detecting time and a first measuring time) given under each of the detecting conditions and a slope varying time which is obtained based on the measure takes an extremely large value in some cases. In other words, a specimen is determined to be negative by the measuring method in some cases. The foregoing cases, in which statistical comparison using numerical values cannot be made between a slope varying time and a measurement result, are removed from observations.

As a result of a t-test conducted on the detecting condition #101 in Fig. 4 and the measure thereof, it is appreciated that a probability for a critical region is smaller than 1 % (=0.01). Also, the absolute value of t is larger than the t critical. Accordingly, it is appreciated that there is a significant difference between a mean of slope varying times and a mean of measuring times each required for an oxygen current to reach the threshold value Ith in the conventional arts. Thus, as described above in the paragraphs entitled "A. Basic Concept of the Present Invention", it is possible to calculate the initial number of bacteria earlier by obtaining a slope varying time of an oxygen current.

Meanwhile, by referring to Table 5 and Table 6, a null hypothesis that there is no significant difference between a mean of first measuring times respectively obtained under the detecting conditions #319, #326, and #329 and a mean of slope varying times respectively paired with the foregoing first measuring times is not rejected, on one hand. On the other hand, it can be concluded that, with respect to the other detecting conditions than the detecting conditions #319, #326, and #329, the foregoing null hypothesis can be rejected and there is a significant difference. Although a null hypothesis cannot be validated because of non-rejectability of the null hypothesis in a strict sense, such a conclusion as noted above is employed in many cases in general. Thus, it is determined that only each of the means of the first measuring times respectively obtained under the detecting conditions #319, #326, and #329 out of the various detecting conditions shown in Table 1, Table 2, and Table 3 does not significantly differ from an average value of slope varying times which are obtained by visual check.

Now, it is expected that to employ the detecting conditions #319, #326, and #329 makes it possible to replace a slope varying time obtained by visual check by a first measuring time which allows automatic operations as described in the first preferred embodiment and the second preferred embodiment. Then, a first measuring time in each of the detecting conditions #319, #326, and #329 was compared with a first measuring time in the detecting condition #101. Table 7 shows results of t-tests (significance level of 1 %) conducted on means of measuring times, and Table 8 shows results of t-tests (significance level of 5 %) conducted on means of coefficients of variation (CV values) of measuring times. Also those results were subjected to significant difference tests by carrying out a one-sample t-test in the same manner as the results in Table 4, Table 5, and Table 6.

**[Table 7]**

| | #101 | #319 | #101 | #326 | #101 | #329 |
|---|---|---|---|---|---|---|
| Mean | 556.56 | 499.64 | 544.02 | 481.77 | 552.48 | 502.35 |
| Variance | 4.40E+04 | 2.60E+04 | 4.97E+04 | 2.98E+04 | 4.54E+04 | 2.85E+04 |
| Observations | 259 | 259 | 260 | 260 | 254 | 254 |
| Degrees of Freedom | 258 | | 259 | | 253 | |
| t | 7.40 | | 6.97 | | 6.13 | |
| P(T<=t) two-tail | 1.91527E-12 | | 2.65675E-11 | | 3.34619E-09 | |
| t Critical two-tail | 2,60 | | 2.59 | | 2.60 | |

**[Table 8]**

| | #101 | #319 | #101 | #326 | #101 | #329 |
|---|---|---|---|---|---|---|
| Mean | 12.27 | 9.52 | 12.12 | 11.27 | 12.22 | 9.74 |
| Variance | 192.19 | 70.10 | 187.62 | 133.32 | 193.09 | 72.79 |
| Observations | 70 | 70 | 72 | 72 | 70 | 70 |
| Degrees of Freedom | 69 | | 71 | | 69 | |
| t | 1.91 | | 0.60 | | 1.77 | |
| P(T<=t) two-tail | 0.06034342 | | 0.552873319 | | 0.080389151 | |
| t Critical two-tail | 2.00 | | 2.00 | | 2.00 | |

It is appreciated from Table 7 that there is a significant difference between a mean of the first measuring times in each of the detecting conditions #319, #326, and #329 and a mean of measuring times in the detecting condition #101. Accordingly, the first measuring time in each of the detecting conditions #319, #326, and #329, like a slope varying time obtained by visual check, produces the effect of shortening a time as compared to a time required for an oxygen current to reach the threshold value Ith in the conventional arts.

Also, by referring to Table 8, a null hypothesis that there is no significant difference between a CV value in each of the detecting conditions #319 and #329 and a CV value in the detecting condition #101 cannot be rejected. Based on this, it was determined that the first measuring times in the detecting conditions #319 and #329 vary to the same degree as the measuring times in the conventional arts. Further, it was also determined that the detecting condition #326 is not effective in view of variation.

Next, a determination was made as to whether bacteria in each of all the 358 specimens was positive or negative under each of the four detecting conditions #101, #319, #326, and #329. Then, the results of the determinations were compared with results of determinations as to whether bacteria are positive or negative, which determinations are made by a method using an agar medium enrichment and incubation are caused (which method will hereinafter be referred to as an "agar method"). The results of the comparison are shown in Table 9.

**[Table 9]**

| All of 358 Specimens | Number of False-Positive Specimens | Number of False-Negative Specimens | Probability of Coincidence With Agar Method |
|---|---|---|---|
| #101 | 5 | 25 | 91.6% |
| #319 | 14 | 45 | 83.5% |
| #326 | 14 | 45 | 83.5% |
| #329 | 14 | 49 | 82.4% |

It is noted that the term "false-positive" is used to indicate a case in which a specimen determined to be positive under each of the detecting conditions is determined to be negative by an agar method. Also, the term "false-negative" is used to indicate a case in which a specimen determined to be negative under each of the detecting conditions is determined to be positive by an agar method. Thus, each of the terms is used to indicate a case in which a result is different from a result given by an agar method. Also, the number of specimens which are determined to be "false-positive" and the number of specimens which are determined to be "false-negative", together with the probability of coincidence with a result given by an agar method, are shown per detecting condition in Table 9. For example, under the detecting condition #101, the numbers of samples which are determined to be "false-positive" and "false-negative" are 5 and 25, respectively, so that the probability of coincidence is (358-5-25)/358 × 100 = 91.6 (%).

The results from Table 9 shows lower probability of coincidence with an agar method compared to the detecting condition #101 according to the conventional arts. Thus, improvement to further increase the probability of coincidence with an agar method was made while following a basic concept of the detecting method (in which the flow chart in Fig. 4 is employed as the step SB) for each of the detecting conditions #326 and #329. Specifically, not the flow chart in Fig. 7, but the flow chart in Fig. 6 was employed as the step SC, so that 0.8 was employed as the coefficient q. Also, 0.6 (nA/mm²/min²) was employed as the preset value E1. Further, the preset values Z1 and Z2 were assumed to be five and ten, respectively. The foregoing detecting condition is identified by the number of "#331". A dead time was assumed to be 200 minutes. Furthermore, a slope of an oxygen current was obtained by the least squares method, to correspond to the slope Knew in the step S107 (refer to Fig. 3).

Moreover, in addition to the detecting condition #331, a detecting condition #332 was set by further employing the second threshold value Id described above in the third preferred embodiment. More specifically, the flow charts shown in Figs. 6, 13, 14, and 16 (the steps S111a and S 114a are employed in the flow chart in Fig. 16) are employed in the detecting condition #332. The second threshold value Id is assumed to be 60 nA/mm², and a measuring time obtained under the detecting condition #332 indicates both a first measuring time and a second measuring time (according to the third preferred embodiment).

Then, results given under each of the detecting conditions #331 and #332 were compared to results given under the detecting condition #101. Table 10 shows results of t-tests (significance level of 1 %) conducted on means of measuring times, and Table 11 shows results of t-tests (significance level of 5 %) conducted on average values of CV values of measuring times. Also those results were subjected to a significant difference test by carrying out a one-sample t-test in the same manner as the results shown in Table 7 and Table 8.

**[Table 10]**

| | #101 | #331 | #101 | #332 |
|---|---|---|---|---|
| Mean | 531.29 | 498.1 5 | 512.95 | 483.81 |
| Variance | 5.20E+04 | 3.05E+04 | 5.77E+04 | 3.38E+04 |
| Observations | 275 | 275 | 287 | 287 |
| Degrees of Freedom | 274 | | 286 | |
| t | 5.23 | | 4.74 | |
| P(T<=t) two-tail | 3.43341E-07 | | 3.40042E-06 | |
| t Critical two- tail | 2.59 | | 2.59 | |

**[Table 11]**

| | #101 | #331 | #101 | #332 |
|---|---|---|---|---|
| Mean | 12.27 | 9.38 | 12.27 | 9.44 |
| Variance | 192.19 | 81.28 | 192.19 | 80.46 |
| Observations | 70 | 70 | 70 | 70 |
| Degrees of Freedom | 69 | | 69 | |
| t | 2.37 | | 2.32 | |
| P(T < =t) two- tail | 0.020713609 | | 0.02329029 | |
| t Critical two-tail | 1.99 | | 1.99 | |

It is appreciated from Table 10 that there is a significant difference between a mean of measuring times in each of the detecting conditions #331 and #332 and a mean of measuring times in the detecting condition #101. Accordingly, the measuring times in the detecting conditions #331 and #332, like the measuring times in the detecting conditions #319, #326, and #329, produce the effect of shortening a time as compared to a measuring time required for an oxygen current to reach the threshold value Ith in the conventional arts.

It is also appreciated from Table 11 that there is a significant difference about a CV value between each of the detecting conditions #331 and #332 and the detecting condition #101. Thus, to employ the detecting conditions #331 and #332 could more effectively improve variation in measuring times as compared to variation in measuring time according to the conventional arts.

In view of the foregoing, a determination was made as to whether bacteria in each of all the 358 specimens was positive or negative under those detecting conditions. Then, the results of the determinations were compared with results of determinations as to whether or not bacteria was positive or negative, which determinations were made by an agar method. The results of comparisons are shown in Table 12.

**[Table 12]**

| All of 358 Specimens | Number of False-Positive Specimens | Number of False-Negative Specimens | Probability of Coincidence With Agar Method |
|---|---|---|---|
| #331 | 11 | 31 | 88.3% |
| #332 | 11 | 19 | 91.6% |

Comparing to Table 9, it is appreciated that even under the detecting condition #331 which does not include a second measuring time, the probability of coincidence with an agar method are improved to be 88.3%. Further, under the detecting condition #332 which includes a second measuring time, the probability of coincidence with an agar method is 91.6%, which is identical to that according to the conventional arts. Moreover, the number of false-negative specimens is smaller than that provided under the conventional detecting condition #101, causing a preferable tendency for safety.

As is made from the above description, it is more preferable to employ the flow chart shown in Fig. 4 than that shown in Fig. 5, as the step SB. Also, it is more preferable to employ the steps S111a and S114a (refer to Fig. 6) than the steps S111b and S114b (refer to Fig. 7) in the step SA, and further, it is preferable to set a dead time (refer to Fig. 13). Moreover, it is more preferable to include a second measuring time (refer to Fig. 16) described in the third preferred embodiment.

While the invention has been shown and described in detail, the foregoing description is in all aspects illustrative and not restrictive. It is therefore understood that numerous modifications and variations can be devised without departing from the scope of the invention.

## Claims

1. A method of measuring the number of microorganisms sequentially measuring (S101, S 102) an oxygen current (Inew) which is a current flowing depending on an amount of oxygen in a culture medium containing microorganisms, the initial number of which is a target of measurement, to obtain a first measuring time (ts) required for the oxygen current to fall below a first threshold value (IP) a preset number of times (Z2), and comprising the steps of:
(a) obtaining a time difference value (Knew) which is a difference in the oxygen current per unit time, from a measured value of the oxygen current in a predetermined measuring period (SA);
(b) determining as to whether or not the time difference value obtained in the step (a) becomes stable (SB); and
(c) when the determination in the step (b) gives a positive result, setting the first threshold value which depends on and is lower than a value based on values of the oxygen current (I1 through IZ+1) resulting from measurements in the latest measuring period (S114a, S114b).

2. The method of measuring the number of microorganisms according to claim 1, wherein
in the step (c), the first threshold value (IP) is set by subtracting a preset threshold value (Δ) from a value of the oxygen current (IZ+1) which has been most recently measured.

3. The method of measuring the number of microorganisms according to claim 1, wherein
in the step (c), the first threshold value (IP) is set by multiplying a value of the oxygen current (IZ+1) which has been most recently measured by a coefficient (q) which is larger than zero and less than one.

4. The method of measuring the number of microorganisms according to claim 3, wherein
the coefficient (q) is set to 0.8.

5. A method of measuring the number of microorganisms sequentially measuring (S101, S102) an oxygen current (Inew) which is a current flowing depending on an amount of oxygen in a culture medium containing microorganisms, the initial number of which is a target of measurement, to obtain a first measuring time (tu) required for a decrease of the oxygen current to be moderated below a threshold value (E3), comprising the steps of:
(a) obtaining a time difference value (Knew) which corresponds to a difference in the oxygen current per unit time, from a measured value of the oxygen current in a predetermined measuring period (SA);
(b) determining as to whether or not the time difference value obtained in the step (a) becomes stable (SB); and
(c) when the determination in the step (b) gives a positive result, determining the measuring time based on the latest measured measuring period if a value of the difference value (Knew) provided by a measurement after the step (b) increases to be larger than a value of the difference value (Kold) provided by a measurement in the step (b) by increments exceeding the threshold value (SC).

6. The method of measuring the number of microorganisms according to any one of claims 1 through 5, wherein
in the step (b), the time difference value is determined to become stable by acknowledging that an absolute value (|(Knew)| of the time difference value falls within a predetermined range (E2) in a predetermined period (S 1 08b, S109).

7. The method of measuring the number of microorganisms according to any one of claims 1 through 5, wherein
in the step (b), the time difference value is determined to become stable by acknowledging that an absolute value of variation in the time difference value with respect to time (|Knew-Kold)|/Δt) falls within a predetermined range (E1) in a predetermined period (S 1 08a, S109).

8. The method of measuring the number of microorganisms according to claim 7, wherein
the predetermined range (E1) is equal to 0.6 nA/mm²/min² in terms of current density,
the preset number of times (Z2) is equal to ten, and
the predetermined period is long enough for the oxygen current to be measured five (Z1) times in succession.

9. The method of measuring the number of microorganisms according to any one of claims 1 through 5, wherein
in the step (b), a determination as to whether or not the time difference value becomes stable is made after a predetermined dead time passes after the step (a).

10. The method of measuring the number of microorganisms according to claim 9, wherein
the predetermined dead time is set to 200 minutes.

11. The method of measuring the number of microorganisms according to any one of claims 1 through 5, further comprising the step of
(d) obtaining a second measuring time (tt) required for the oxygen current to decrease to fall below a second threshold value (Id).

12. The method of measuring the number of microorganisms according to claim 11, wherein
the second threshold value (Id) is equal to 60 nA/mm²/min² in terms of current density.

13. The method of measuring the number of microorganisms according to any one of claims 1 through 5, wherein
based on a calibration curve obtained for the microorganisms and the culture media from a relationship between the first measuring time and the initial number of microorganisms which is known, of each of the culture media of the same kind which contain the microorganisms, the initial numbers of which are known and differ from each other,
the first measuring time is obtained for the culture medium of the same kind which contains the microorganisms, the initial number of which is unknown to thereby calculate the initial number of microorganisms which is unknown.

14. The method of measuring the number of microorganisms according to any one of claims 1 through 5, wherein
the first measuring time is respectively obtained for each of the culture media of the same kind the initial numbers of the microorganisms in which are known and differ from each other, and
the calibration curve for the microorganisms and the culture media is obtained from a relationship between the plural initial numbers of microorganisms which are known and plural of the first measuring time.

15. An apparatus for measuring the number of microorganisms comprising:
an oxygen current measuring part (201) for sequentially measuring (S101, S 112) an oxygen current (Inew) which is a current flowing depending on an amount of oxygen in a culture medium containing microorganisms, the initial number of which is a target of measurement; and
an evaluating part (202) for obtaining a first measuring time (ts) required for the oxygen current to decrease to fall below a first threshold value (IP) a preset number of times (Z2), wherein
the evaluating part performs the steps of:
(a) obtaining a time difference value (Knew) which is a difference in the oxygen current per unit time, from a measured value of the oxygen current in a predetermined measuring period (SA);
(b) determining as to whether or not the time difference value obtained in the step (a) becomes stable (SB); and
(c) when the determination in the step (b) gives a positive result, setting the first threshold value which depends on and is lower than a value based on values of the oxygen current (I1 through IZ+1) resulting from measurements in the latest measuring period (S 114a, S 114b).

16. The apparatus for measuring the number of microorganisms according to claim 15, wherein
in the step (c), the first threshold value (IP) is set by subtracting a preset threshold value (Δ) from a value of the oxygen current (IZ+1) which has been most recently measured.

17. The apparatus for measuring the number of microorganisms according to claim 15, wherein
in the step (c), the first threshold value (IP) is set by multiplying a value of the oxygen current (IZ+1) which has been most recently measured by a coefficient (q) which is larger than zero and less than one.

18. The apparatus for measuring the number of microorganisms according to claim 17, wherein
the coefficient (q) is set to 0.8.

19. An apparatus for measuring the number of microorganisms comprising:
an oxygen current measuring part (201) for sequentially measuring (S101, S102) an oxygen current (Inew) which is a current flowing depending on an amount of oxygen in a culture medium containing microorganism, the initial number of which is a target of measurement; and
an evaluating part (202) for obtaining a first measuring time (tu) required for a decrease of the oxygen current to be moderated below a threshold value (E3), wherein
the evaluating part performs the steps of:
(a) obtaining a time difference value (Knew) which is a difference in the oxygen current per unit time, from a measured value of the oxygen current in a predetermined measuring period (SA);
(b) determining as to whether or not the time difference value obtained in the step (a) becomes stable (SB); and
(c) when the determination in the step (b) gives a positive result, determining the measuring time based on the latest measured measuring period if a value of the difference value (Knew) provided by a measurement after the step (b) increases to be larger than a value of the difference value (Kold) provided by a measurement in the step (b) by increments exceeding the threshold value (SC).

20. The apparatus for measuring the number of microorganisms according to any one of claims 15 through 19, wherein
in the step (b), the time difference value is determined to become stable by acknowledging that an absolute value (|(Knew)| of the time difference value falls within a predetermined range (E2) in a predetermined period (S108b, S109).

21. The apparatus for measuring the number of microorganisms according to any one of claims 15 through 19, wherein
in the step (b), the time difference value is determined to become stable by acknowledging that an absolute value of variation in the time difference value with respect to time (|Knew - Kold|/Δt) falls within a predetermined range (E1) in a predetermined period (S108a, S109).

22. The apparatus for measuring the number of microorganisms according to claim 21, wherein
the predetermined range (E1) is equal to 0.6 nA/mm²/min² in terms of current density,
the preset number (Z2) of times is equal to ten, and
the predetermined period is long enough for the oxygen current to be measured five (Z1) times in succession.

23. The apparatus for measuring the number of microorganisms according to any one of claims 15 through 19, wherein
in the step (b), a determination as to whether or not the time difference value becomes stable is made after a predetermined dead time passes after the step (a).

24. The apparatus for measuring the number of microorganisms according to claim 23, wherein
the predetermined dead time is set to 200 minutes.

25. The apparatus for measuring the number of microorganisms according to any one of claims 15 through 19, wherein
the evaluating part (201) further performs a step of
(d) obtaining a second measuring time (tt) required for the oxygen current to decrease to fall below a second threshold value (Id).

26. The apparatus for measuring the number of microorganisms according to claim 25, wherein
the second threshold value (Id) is equal to 60 nA/mm²/min² in terms of current density.

27. The apparatus for measuring the number of microorganisms according to any one of claims 15 through 19, wherein
based on a calibration curve obtained for the microorganisms and the culture media from a relationship between the first measuring time and the initial number of microorganisms which is known, of each of the culture media of the same kind which contain the microorganisms, the initial numbers of which are known and differ from each other,
the first measuring time is obtained for the culture medium of the same kind which contains the microorganisms, the initial number of which is unknown to thereby calculate the initial number of microorganisms which is unknown.

28. The apparatus for measuring the number of microorganisms according to any one of claims 15 through 19, wherein
the first measuring times is respectively obtained for each of the culture media of the same kind, the initial numbers of the microorganisms in which are known and differ from each other, and
the calibration curve for the microorganisms and the culture media is obtained from a relationship between the plural initial numbers of microorganisms which are known and plural of the first measuring time.
